# EUROPEAN PATENT SPECIFICATION

(11) **EP 1 817 058 B1**
(45) Date of publication and mention of the grant of the patent: **02.09.2015**
(21) Application number: 05852254.1
(22) Date of filing: 23.11.2005
(51) Int. Cl.: A61K 47/00, A23K 1/16, A23K 1/18

(54) **COMPOSITION FOR USE IN IMPROVING LIVER CLEARANCE OF XENOBIOTIC SUBSTANCES IN AN ANIMAL**
ZUSAMMENSETZUNG ZUR VERWENDUNG DER VERBESSERUNG DER BESEITIGUNG VON XENOBIOTISCHEN SUBSTANZEN AUS DER LEBER BEI EINEM TIER
COMPOSITION DESTINEE A ETRE UTILISEE POUR FACILITER LE DECHARGEMENT DU FOIE D'UN ANIMAL DES SUBSTANCES XENOBIOTIQUES

(30) Priority: 24.11.2004 US 630971 P
(43) Date of publication of application: 15.08.2007
(73) Proprietor: Hill's Pet Nutrition, Inc., Topeka, KS 66603 (US)
(72) Inventor: ZICKER, Steven, Curtis, Lawrence, Kansas 66049 (US); PAETAU-ROBINSON, Inke, Auburn, Kansas 66402 (US)
(74) Representative: Daniels, Jeffrey Nicholas
(86) International application number: PCT/US2005/042886
(87) International publication number: WO 2006/058278

(56) References cited:
- US-A- 5 624 896
- US-A1- 2002 006 907
- US-A1- 2002 076 469
- US-A1- 2002 076 469
- US-A1- 2003 224 061
- US-B2- 6 914 071
- Beth M. Ley: "Alpha Lipoic Acid", , 14 June 2000 (2000-06-14), XP002676684, Retrieved from the Internet: URL:http://web.archive.org/web/20000614235 007/http://www.tldp.com/issue/160/160alpha .htm [retrieved on 2012-05-25]
- BERKSON B M: "A conservative triple antioxidant approach to the treatment of hepatitis C. Combination of alpha lipoic acid (thioctic acid), silymarin, and selenium: three case histories", MEDLINE,, 15 October 1999 (1999-10-15), XP002567154,

## Description

### CROSS-REFERENCE TO RELATED APPLICATIONS

This application claims priority to U.S. Provisional Application Serial No. 60/630,971, filed November 24, 2004, the disclosure of which is incorporated herein by this reference.

### BACKGROUND OF THE INVENTION

### Field of the Invention

The present invention generally relates to methods for improving animal health and particularly to methods for improving liver clearance of xenobiotic substances.

### Description of the Related Art

The liver is a vital organ and has an important role in most every bodily function of a mammal. In one role, the liver acts as a filtration system to protect other organs from the effects of toxin buildup. Toxins absorbed from the digestive system are removed from the blood by the liver before they can affect the rest of the body. The capacity of a xenobiotic such as a drug, therapeutic agent, or chemical to produce injury to a liver is known as hepatotoxicity. The xenobiotic is a pharmacologically or toxicologically active substance not indigenously produced and therefore foreign to an organism. Many industrial compounds, drugs and other therapeutic agents are well established as injurious to a liver. As mammals age, their capacity for the filtration and clearance of xenobiotics by the liver decreases. It is well known that as mammals age, especially companion animals, they encounter health problems that require drugs and other therapeutic agents. Since liver filtration and clearance decreases in such an aged animal, administration of such drugs and therapeutic agents to improve the health of the animal may have hepatotoxic effects. What is needed are methods that improve xenobiotic filtration and clearance by the liver in aging companion animals.

US2002/0006907 discloses an alpha-lipoic acid-based food supplement for increasing lean muscle mass and strength.

US2003/0224061 discloses an oral composition with insulin-like activities.

Beth M. Ley 2000 (XP002676684) is directed to the antioxidant properties of lipoic acid.

### SUMMARY OF THE INVENTION

The present invention provides a composition comprising lipoic acid for use in improving liver clearance of xenobiotic substances in an animal, wherein the use comprises feeding the composition to the animal.

The present invention further provides a use of a composition that comprises lipoic acid to prepare a medicament for improving liver clearance of xenobiotic substances in an animal.

In various embodiments, the invention is a new approach for improving the health of aging animals, especially dogs, based upon the use of lipoic acid as part of a diet that is fed to the animals.

Further areas of applicability of the present invention will become apparent from the detailed description provided hereinafter. It should be understood that the detailed description and specific examples, while indicating the illustrative embodiments of the invention, are not intended to limit the scope of the invention.

### BRIEF DESCRIPTION OF THE DRAWINGS

The present invention will become more fully understood from the detailed description and the accompanying drawings.

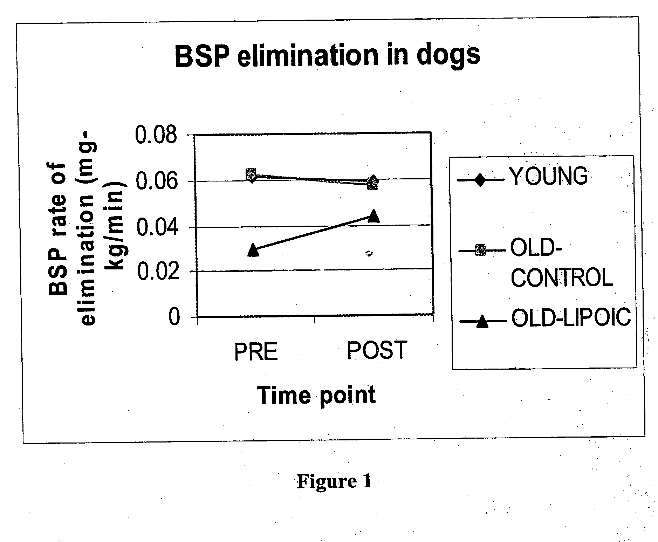

Figure 1 is a graphical representation of the effect of lipoic acid on liver clearance in older dogs as compared to young dogs and older control dogs.

The Figure is intended to exemplify the general characteristics of the invention for the purposes of the description of such embodiments herein. The Figure may not precisely reflect the characteristics of any given embodiment and is not necessarily intended to define or limit specific embodiments within the scope of this invention.

### DETAILED DESCRIPTION OF THE INVENTION

### Definitions

The term "animal" means any animal susceptible to or suffering from impaired liver function and in need of improved liver clearance of xenobiotic substances or an animal that could benefit from improved liver clearance of xenobiotic substances. An animal is "susceptible to" a disease or condition if the animal exhibits symptoms that indicate that the animal is likely to develop the condition or disease. An animal is "suffering from" a disease or condition if the animal exhibits symptoms that are indicative that the animal has developed the condition or disease.

The term "older animal" means any animal susceptible to or suffering from impaired liver function and in need of improved liver clearance of xenobiotic substances or an animal that could benefit from improved liver clearance of xenobiotic substances because of age.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

### The Invention

In one aspect, the present invention provides compositions comprising lipoic acid for use in improving liver clearance of xenobiotic substances in animals. The use comprises feeding the composition comprising lipoic acid to the animal. Generally, the lipoic acid is feed to the animal in amounts of greater than 5 mg per day, preferably from about 10 to about 1000 mg per day, most preferably from about 50 to about 500 mg per day. The compositions comprise lipoic acid in amounts of at least 50 ppm, preferably at least 150 ppm. In various embodiments, the methods and compositions are useful for improving liver clearance of xenobiotic substances in animals, particularly in older animals.

The nutrition and health of animals is one of the most important aspects of care, particularly pet care for companion animals. Many caregivers have a difficult time determining if an animal is receiving a well-balanced and healthy diet. While people are becoming much more aware regarding their own personal nutrition, there is little knowledge of the advanced dietary requirements that an animal must have.

The present description provides a method for feeding an animal, e.g., a companion animal such as a dog, a composition or diet containing lipoic acid to enhance hepatic function, particularly when it may be impaired by age, and improve the overall health of the animal. The amount of lipoic acid given to the animal is a non-toxic amount. The lipoic acid may be provided to the animal either as a supplement or contained in a composition, including a diet, fed to the animal. Such a supplement may be in the form of a pill or capsule, a treat or a biscuit, or any other edible form. By "diet", it is meant the food or drink regularly consumed by the animal. A diet may include supplements consumed by the animal. A diet is considered to have essentially enough nutrients to be life sustaining for the animal. A companion animal diet can be any suitable pet food formula which also provides adequate nutrition for the animal. For example, a typical canine diet for use in the present invention may contain from about 8 to 50% fat, about 16 to 50% by weight protein and about 3 to 15% total dietary fiber. In another example, a typical feline diet may contain from about 8 to 50% by weight fat, and from about 30 to 60% by weight protein. However, no specific ratios or percentages of these or other nutrients are required. A nutrient is any food constituent that helps support life. Nutrients important to an animal's health are known to skilled artisans, e.g., proteins, carbohydrates, fats, fibers, vitamins, and minerals. Water is also vital to an animal's health.

The free radical theory of aging proposes that oxidative stress results in aging and a decrease in the reduced to oxidized ratio of intercellular anti-toxins such as glutathione. Glutathione is prevalent in the liver and is utilized to conjugate xenobiotics for the elimination into the bile duct and eventual elimination via the feces. As such, decreased ability of glutathione in aged animals may result in the impaired clearance of xenobiotics that contribute to cancer, toxicity and other unwanted effects. In addition, it is known that liver function undergoes senescence with increasing age. Previous studies have shown that GSH:GSSG ratios may be improved in lymphocytes from dogs supplemented with lipoic acid, however, a functional outcome has not been quantified. (See Zicker, SC et al., Veterinary Therapeutics, 3(2):167-176, 2002.)

R-α-lipoic acid (CAS number 1200-22-2, also known as thioctic acid and 1, 2-dithiolane-3-pentanoic acid) naturally occurs in plant and animal tissues, where it is covalently bound to an ε-amino group of lysine residues. Lipoic acid is commercially available and is produced by companies such as BASF and Cognis. Lipoic acid is commercially available as an essentially pure R-α lipoic acid or as a racemic mixture of lipoic acid isomers. In plants, lipoic acid is most abundant in spinach and potatoes while in animal tissues, lipoic acid is most abundant in the kidney and the heart. R-α-lipoic acid was first discovered in 1937 (See Snell et al., Journal Bact. 33; 207, 1937) and was not isolated and characterized until 1951 (See Reed et al. Science 114:94-4, 1951). R-α-lipoic-acid may be synthesized and such methods are well known in the art. (See U.S. Patent No. 2,890,716 to Reed issued April 18, 1961). R-α-lipoic acid has been classified as an antioxidant and has been used in high dosages as a treatment for Type II diabetes. Studies have shown that mixtures of carnitine and lipoic acid may enhance metabolism and alleviate oxidative stress. (See U.S. Patent No. 5,916,912 to Ames et al. issued June 29, 1999 and U.S. Patent No. 6,365,622 to Cavayzo issued April 2, 2002). In addition, it has been shown that a companion animal diet comprising lipoic acid among other ingredients appears to inhibit the deterioration of the mental capacity of an aged companion animal. (See U.S. Patent Application Nos. 20020076469, 20020052402, 20020076470, 2000115710, and 20020119182.)

Studies have shown that mitochondrial oxidation plays a role in the metabolism of lipoic acid. Although the metabolism in humans mainly resembles that observed in mice and rats, the formation of oxidized structures related to tetranorlipoic acid found in canines appears to have no equivalent in humans. In addition, 3-ketolipoic acid, an intermediate in the mitochondrial oxidation of lipoic acid has been reported in plasma samples from rats and humans but has not been found in plasma from canines. (See Schupke, H. et al. Drug Metabolism and Disposition, 29 (6) 855-862, 2001). It appears that the metabolic pathway of α-lipoic acid is different in canines as compared to humans.

Mercapturic acids are sulfur derivatives of N-acetyl-cysteine, which is synthesized from glutathione (GSH). It is generally accepted that most compounds are metabolized to mercapturic acids first undergo conjugation with GSH catalyzed by an enzyme called glutathione S-transferase, found in the soluble or supertant liver refractions. The mercapturic acid pathway appears to have evolved as a protective mechanism against xenobiotic induced heptotoxicity or carcinogenicity, serving to detoxify a large number of noxious substances that are inhaled, ingested or normally produced metabolically every day. Lipoic acid not only up regulates the glutathione but also up regulates the enzyme, glutathione S-transferase, that conjugated glutathione in the liver. Bromosulfophthalein (CAS number 71-67-0 also known as BSP and sulfobromophthalein) is an organic dye that, when injected into the circulation, is removed by the liver at a rate that reflects the liver's ability to extract and metabolize a number of organic compounds. See S.M. Rosenthal, E.C. Wjite, J. Pharmacol. 24,265 (1924) W. Häcki et al., J. Lab. Clin. Med. 88, 1019 (1976). BSP is cleared from the liver in three steps. First, BSP is transferred from albumin through the plasma to the liver. This step is dependent on plasma protein concentration and other ligands that bind to plasma proteins. Secondly, BSP is complexed in the liver by a ligandin and z protein. Finally, BSP is conjugated by glutathione via glutathione S-transferase enzyme and eliminated into the bile duct and this is the rate limiting step. Thus BSP is an example of a xenobiotic that, when measured in the blood after injection, provides information on the functional capabilities of the liver.

Various embodiments of the invention include compositions for use in improving liver clearance of xenobiotic substances in an animal, particularly a companion animal. In such embodiments, the use comprises feeding to the animal a composition, e.g., a diet, comprising lipoic acid in an amount of at least 50 ppm on a dry matter basis. In other embodiments the use comprises feeding to the animal a diet comprising lipoic acid in an amount of at least 100 ppm on a dry matter basis. In still other embodiments, the use comprises feeding to the animal a diet comprising lipoic acid in an amount from about 75 ppm to about 150 ppm on a dry matter basis. As used herein, lipoic acid is in a racemic mixture, but other embodiments may include lipoic acid which is essentially pure R-α lipoic acid or as a lipoate derivative, mixtures of isomers, salts, esters, amides or combinations thereof (For example see US Patent No 5,621,177 to Bethge et al. issued April 15, 1997).

In various embodiments, a composition or diet comprising at least 50 ppm of lipoic acid increases hepatic function in older dogs. In some embodiments, the lipoic acid is added to the companion animal's food. In such embodiments, the lipoic acid may be added during the processing of the companion animal food that is then packaged and made available to consumers. Such processes may include extrusion, canning, baking and the like or any other method or process of producing pet foods that is known in the art. In such processes, the lipoic acid may be contributed by a natural source like an animal or plant component, such as kidney or spinach or the lipoic acid may be contributed, by a synthetically derived source, or the lipoic acid may be contributed by a mixture of natural and synthetic sources. In other embodiments, lipoic acid may be in a capsule form to be fed to the companion animal. In still other embodiments, the lipoic acid may be in a powder or in a crystalline which may be added to the animal's food or fed directly to the animal. In various embodiments, the companion animal diet comprises lipoic acid and other needed nutritional components. In various embodiments, the companion animal is a dog and in other embodiments, the companion animal is a cat. Studies have shown that lipoic acid may be ten times more toxic in cats than in dogs. (See Hill, AS et al., J. Anim. Physiol. Anim. Nutr. 88(3-4): 150-156, 2004). In various embodiments wherein the companion animal is a cat, the diet comprises less than 30 ppm of lipoic acid on a dry weight basis.

In a further aspect, the present invention provides for a use of lipoic acid to prepare a medicament. In another, the invention provides for the use of lipoic acid to prepare a medicament for improving liver clearance of xenobiotic substances in an animal. A xenobiotic substance liver clearance improving amount of lipoic acid may be used to the animal. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

In a further aspect, the present description provides kits suitable for feeding lipoic acid to an animal. The kits comprise in separate containers in a single package or in separate containers in a virtual package, as appropriate, lipoic acid and at least one of (1) one or more ingredients suitable for consumption by an animal, (2) instructions for how to combine the lipoic acid and other kit components to improve liver clearance of xenobiotic substances, particularly to produce a composition useful for improving liver clearance of xenobiotic substances, and (3) instructions for how to use the lipoic acid and other components of the present invention, particularly for the benefit of the animal. When the kit comprises a virtual package, the kit is limited to instructions in a virtual environment in combination with one or more physical kit components. The kit contains the lipoic acid and other components in amounts sufficient to improve liver clearance of xenobiotic substances. Typically, the lipoic acid and the other suitable kit components are admixed just prior to consumption by an animal. In one aspect, the kit contains a packet containing lipoic acid and a container of food for consumption by an animal. The kit may contain additional items such as a device for mixing the lipoic acid and ingredients or a device for containing the admixture, e.g., a food bowl. In another aspect the lipoic acid is mixed with additional nutritional supplements such as vitamins and minerals that promote good health in an animal.

In another aspect, the present description provides a means for communicating information about or instructions for one or more of (1) using lipoic acid to improve liver clearance of xenobiotic substances, (2) admixing lipoic acid with the other components of the present invention, (3) feeding lipoic acid to an animal, alone or in combination with the other elements of the present invention, and (4) using the kits of the present invention for improving liver clearance of xenobiotic substances comprising a document, digital storage media, optical storage media, audio presentation, or visual display containing the information or instructions. In certain aspects, the communicating means comprises a document, digital storage media, optical storage media, audio presentation, or visual display containing the information or instructions. Preferably, the communication means is a displayed web site or a brochure, product label, package insert, advertisement, or visual display containing such information or instructions. Useful information includes one or more of (1) methods and techniques for combining and feeding the lipoic acid and/or other components and (2) contact information for animals or their caregivers to use if they have a question about the invention and its use. Useful instructions include amounts for mixing and administration amounts and frequency. The communication means is useful for instructing on the benefits of using the present invention and communicating the approved methods for feeding the invention to an animal.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. The terms "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents, patent applications, and publications mentioned herein are incorporated herein by reference to the extent allowed by law for the purpose of describing and disclosing the compositions, compounds, methods, and similar information reported therein that might be used with the present invention. However, nothing herein is to be construed as an admission that the invention is not entitled to antedate such disclosure by virtue of prior invention.

### EXAMPLES

This invention can be further illustrated by the following examples of preferred embodiments thereof, although it will be understood that these examples are included merely for purposes of illustration and are not intended to limit the scope of the invention unless otherwise specifically indicated.

### Example 1

The study involves three groups of dogs: Group 1 - young dogs on a controlled food, Group 2 - old dogs on a controlled food, and Group 3 - old dogs on a dry food fortified with 150 ppm of lipoic acid on a dry matter basis. The dogs are beagles and Group 1 consists of 10 beagles with the average age of 5.1 years old, Group 2 consists of 10 beagles with an average age of 11.8 years old, and Group 3 consists of 10 beagles with an average age of 11.3 years old. The dogs from all three groups are fed the controlled food for a two week period prior to intervention. After a two week period, Group 3 is transferred to a diet of dry food fortified with 150 ppm of lipoic acid on a dry matter basis. During the two week period on the control, samples are taken from all dogs and a bromosulthophthalein (BSP) test is administered. BSP is taken up by the liver and conjugated with GSH for elimination in bile duct secretions. The BSP test is well known as a diagnostic test in veterinary medicine to test the functional capability of the liver. The dogs of Group 3 have impaired BSP clearance compared to controls thus are examples of canines with senescent liver function. The three groups of dogs then eat their respective diets for a six week period of time and after this period, the BSP test is administered for a second time. Results as in Figure 1 show that dogs in Group 3 which were all older and included a diet with 150 ppm lipoic acid in a dry matter basis have improved liver clearance of a BSP.

In the specification, there have been disclosed typical preferred embodiments of the invention and, although specific terms are employed, they are used in a generic and descriptive sense only and not for purposes of limitation, the scope of the invention being set forth in the following claims. Obviously many modifications and variations of the present invention are possible in light of the above teachings. It is therefore to be understood that within the scope of the appended claims the invention may be practiced otherwise than as specifically described.

## Claims

1. A composition comprising lipoic acid for use in improving liver clearance of xenobiotic substances in an animal, wherein the use comprises feeding the composition to the animal.

2. The composition for use according to Claim 1, wherein the use comprises feeding the lipoic acid in amounts of greater than 5 mg per day.

3. The composition for use according to Claim 1 wherein the use comprises feeding the lipoic acid in amounts of from about 10 to about 1000 mg per day.

4. The composition for use according to Claim 1 wherein the animal is a companion animal, and preferably, wherein the companion animal is a canine.

5. The composition for use according to Claim 1 wherein the lipoic acid is in capsule form or in powder form.

6. The composition for use according to Claim 1 wherein the lipoic acid is in crystalline form.

7. The composition for use according to Claim 1 wherein the lipoic acid is part of the animal's daily diet, and preferably, wherein the daily diet comprises lipoic acid in an amount of greater than 50 ppm on a dry weight basis.

8. The composition for use according to Claim 1, wherein the lipoic acid is fed to the animal in a food composition suitable for consumption by the animal.

9. The composition for use according to Claim 1; wherein the animal is an older animal.

10. A composition for use according to claim 1, wherein the composition comprises:
a life sustaining amount of nutrients; and
greater than 50 ppm of lipoic acid.

11. The composition for use according to Claim 1 wherein the composition is a food composition.

12. The composition for use according to Claim 11 wherein the animal is a companion animal.

13. The composition for use according to Claim 12 wherein the animal is a canine.

14. The composition for use according to Claim 11 wherein the composition is extruded or canned.

15. A use of a composition that comprises lipoic acid to prepare a medicament for improving liver clearance of xenobiotic substances in an animal.

## Patentansprüche

1. Zusammensetzung, die Liponsäure zur Verwendung beim Verbessern der Beseitigung von xenobiotischen Substanzen aus der Leber eines Tiers umfasst, wobei die Verwendung das Zuführen der Zusammensetzung dem Tier beinhaltet.

2. Zusammensetzung zur Verwendung nach Anspruch 1, wobei die Verwendung das Zuführen der Liponsäure in Mengen von mehr als 5 mg pro Tag beinhaltet.

3. Zusammensetzung zur Verwendung nach Anspruch 1, wobei die Verwendung das Zuführen der Liponsäure in Mengen von etwa 10 bis etwa 1000 mg pro Tag beinhaltet.

4. Zusammensetzung zur Verwendung nach Anspruch 1, wobei das Tier ein Haustier ist und wobei das Haustier vorzugsweise ein Hund ist.

5. Zusammensetzung zur Verwendung nach Anspruch 1, wobei die Liponsäure in Kapselform oder in Pulverform vorliegt.

6. Zusammensetzung zur Verwendung nach Anspruch 1, wobei die Liponsäure in kristalliner Form vorliegt.

7. Zusammensetzung zur Verwendung nach Anspruch 1, wobei die Liponsäure Teil der täglichen Nahrung des Tiers ist und wobei die tägliche Nahrung vorzugsweise Liponsäure in einer Menge von mehr als 50 ppm auf Trockengewichtsbasis umfasst.

8. Zusammensetzung zur Verwendung nach Anspruch 1, wobei die Liponsäure dem Tier in einer für den Verbrauch durch das Tier geeigneten Futterzusammensetzung zugeführt wird.

9. Zusammensetzung zur Verwendung nach Anspruch 1, wobei das Tier ein älteres Tier ist.

10. Zusammensetzung zur Verwendung nach Anspruch 1, wobei die Zusammensetzung Folgendes umfasst:
eine lebenserhaltende Menge an Nährstoffen; und
mehr als 50 ppm Liponsäure.

11. Zusammensetzung zur Verwendung nach Anspruch 1, wobei die Zusammensetzung eine Futterzusammensetzung ist.

12. Zusammensetzung zur Verwendung nach Anspruch 11, wobei das Tier ein Haustier ist.

13. Zusammensetzung zur Verwendung nach Anspruch 12, wobei das Tier ein Hund ist.

14. Zusammensetzung zur Verwendung nach Anspruch 11, wobei die Zusammensetzung extrudiert oder konserviert ist.

15. Verwendung einer Zusammensetzung, die die Liponsäure umfasst, zur Herstellung eines Medikaments zum Verbessern der Beseitigung von xenobiotischen Substanzen aus der Leber eines Tiers.

## Revendications

1. Composition comprenant de l'acide lipoïque pour une utilisation dans l'amélioration de la clairance hépatique de substances xénobiotiques chez un animal, l'utilisation comprenant l'administration de la composition à l'animal.

2. Composition pour une utilisation selon la revendication 1, l'utilisation comprenant l'administration de l'acide lipoïque en quantités supérieures à 5 mg par jour.

3. Composition pour une utilisation selon la revendication 1, l'utilisation comprenant l'administration de l'acide lipoïque suivant des quantités allant d'environ 10 à environ 1000 mg par jour.

4. Composition pour une utilisation selon la revendication 1, l'animal étant un animal de compagnie, et de préférence l'animal de compagnie étant un canidé.

5. Composition pour une utilisation selon la revendication 1, l'acide lipoïque se présentant sous forme de capsule ou sous forme de poudre.

6. Composition pour une utilisation selon la revendication 1, l'acide lipoïque se présentant sous forme cristalline.

7. Composition pour une utilisation selon la revendication 1, l'acide lipoïque faisant partie du régime alimentaire quotidien de l'animal, et de préférence, le régime alimentaire quotidien comprenant de l'acide lipoïque dans une quantité supérieure à 50 ppm sur une base de poids à sec.

8. Composition pour une utilisation selon la revendication 1, l'acide lipoïque étant administré à l'animal dans une composition alimentaire convenant à une consommation par l'animal.

9. Composition pour une utilisation selon la revendication 1, l'animal étant un animal d'un certain âge.

10. Composition pour une utilisation selon la revendication 1, la composition comprenant :
une quantité d'éléments nutritifs pour entretenir la vie ; et
davantage que 50 ppm d'acide lipoïque.

11. Composition pour une utilisation selon la revendication 1, la composition étant une composition alimentaire.

12. Composition pour une utilisation selon la revendication 11, l'animal étant un animal de compagnie.

13. Composition pour une utilisation selon la revendication 12, l'animal étant un canidé.

14. Composition pour une utilisation selon la revendication 11, la composition étant extrudée ou mise en boîtes.

15. Utilisation d'une composition qui comprend de l'acide lipoïque pour préparer un médicament destiné à améliorer la clairance hépatique de substances xénobiotiques chez un animal.
